(19) Europäisches Patentamt
European Patent Office
Office européen des brevets

(11) **EP 4 582 417 A1**

(12) **EUROPEAN PATENT APPLICATION**
published in accordance with Art. 153(4) EPC

(43) Date of publication:
**09.07.2025 Bulletin 2025/28**

(21) Application number: **23858546.7**

(22) Date of filing: **15.02.2023**

(51) International Patent Classification (IPC):
***C07D 401/12*** (2006.01) ***A61P 35/00*** (2006.01)
***A61K 31/4406*** (2006.01) ***A61K 31/4439*** (2006.01)
***G01N 30/02*** (2006.01) ***G01N 30/06*** (2006.01)

(86) International application number:
**PCT/CN2023/076093**

(87) International publication number:
**WO 2024/045503 (07.03.2024 Gazette 2024/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.08.2022 CN 202211051615**

(71) Applicant: **Shenzhen Chipscreen Biosciences Co., Ltd.**
**Guangdong 518057 (CN)**

(72) Inventors:
- **LI, Zhibin**
  **Shenzhen, Guangdong 518057 (CN)**
- **SHAN, Song**
  **Shenzhen, Guangdong 518057 (CN)**
- **ZHOU, You**
  **Shenzhen, Guangdong 518057 (CN)**
- **YANG, Qianjiao**
  **Shenzhen, Guangdong 518057 (CN)**
- **LU, Xianping**
  **Shenzhen, Guangdong 518057 (CN)**

(74) Representative: **Müller, Christian Stefan Gerd**
**ZSP Patentanwälte PartG mbB**
**Hansastraße 32**
**80686 München (DE)**

(54) **TRKA (G667C) AND FLT3 TARGET INHIBITOR AND COMBINATION OF SAME WITH CHIDAMIDE**

(57) Disclosed is a TRKA (G667C), FLT3 and FLT3 (D835Y) target inhibitor N-[4-(6-fluoro-1H-2-benzimidazolyl)benzyl]-(E)-3-(3-pyridine)acrylamide as shown in formula (I), a preparation method therefor, a pharmaceutical composition containing same and chidamide, and an application thereof. By means of in-depth research, it has been found in the present invention that the inhibitor has excellent inhibitory effects on TRKA (G667C) and FLT3 kinase, and has an unexpected synergistic effect when combined with chidamide. The present invention also provides a use thereof as a reference substance for checking related substances in chidamide tablets to control the quality of the chidamide tablets.

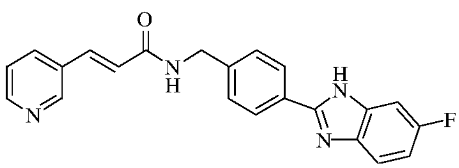

(I)

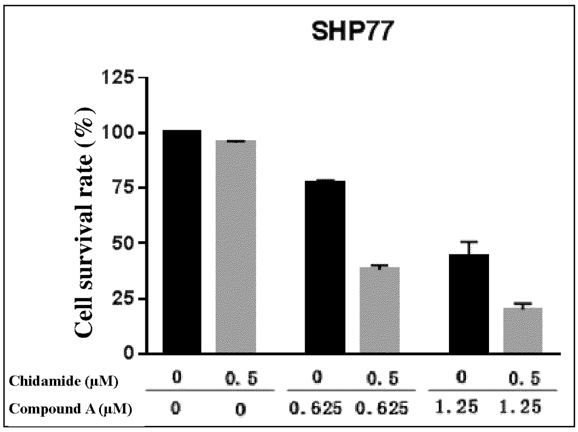


**FIG. 1**

EP 4 582 417 A1

## Description

### FIELD

The present disclosure pertains to the field of pharmaceuticals and particularly relates to a TRKA (G667C) and FLT3 target inhibitor and a composition comprising same and chidamide.

### BACKGROUND

TRKs (tropomyosin-related kinases) are a class of neurotrophin receptors. They belong to the receptor tyrosine kinase family and include three subtypes: TRKA, TRKB, and TRKC, which are encoded by the NTRK1, NTRK2, and NTRK3 genes, respectively. When the NTRK genes fuse with other genes, receptor dimerization and phosphorylation can be induced, leading to high expression of TRK or constant elevation of TRK activity, and the downstream PI3K/Akt/mTOR, PLCγ, and Ras/Raf/MEK/ERK signaling cascade pathways are activated, thereby regulating tumor cell growth, proliferation, invasion, migration, angiogenesis, and drug resistance.

Alterations in TRK signaling pathways mainly include gene fusion, protein overexpression, single nucleotide variations, and splicing mutations. Among these, NTRK gene fusion is the most well-defined oncogenic driver and can be found in various types of adult and pediatric tumors. In patients with congenital nephroma, infantile sarcoma, mammary analog secretory carcinoma (MASC), and secretory breast cancer, the incidence of NTRK gene fusion is over 90%. In patients with papillary thyroid carcinoma, gastrointestinal stromal tumors, and Spitzoid melanoma, the incidence of NTRK gene fusion is between 5% and 25%. In most other tumor patients, the incidence of NTRK gene fusion is less than 5%.

A total of two first-generation TRK inhibitors have been approved: larotrectinib and entrectinib. In 2018, larotrectinib was approved in the United States for the treatment of adult and pediatric patients with locally advanced or metastatic solid tumors carrying NTRK gene fusion. In 2019, entrectinib was approved in the United States for the treatment of adult and pediatric patients with advanced or recurrent solid tumors carrying NTRK gene fusion and patients with metastatic NSCLC carrying an ROS1 rearrangement positive.

However, patients could develop on-target resistance to the first-generation TRK inhibitors. In different cancer types, TRKA G595R, G667C, F589L, etc. and TRKC G623R, G696A, etc. are relatively commonly reported.

FLT3 (FMS-like tyrosine kinase 3) belongs to the class III receptor tyrosine kinase family. Its protein structure contains an extracellular region consisting of five immunoglobulin-like domains, one transmembrane region, one juxtamembrane region, and two intracellular tyrosine kinase regions separated by a kinase insert region. Generally, after the FLT3 ligand binds to FLT3, multiple signaling pathways are activated, including signal transducer and activator of transcription 5 (STAT5), Ras/mitogen-activated protein kinase (MAPK), and phosphoinositide 3-kinase (PI3K)/AKT pathways. FLT3 plays a very important role in the proliferation, differentiation, and apoptosis of hematopoietic cells.

First-generation FLT3 inhibitors include tandutinib, sunitinib, midostaurin, lestaurtinib, sorafenib, etc. As the first-generation FLT3 inhibitors did not initially undergo sensitivity and selectivity screening against the activated FLT3 kinase, second-generation FLT3 inhibitors with higher selectivity and greater inhibitory activity were developed. Among these, gilteritinib, quizartinib, and crenolanib (CP-868596) have been approved for clinical use in Japan, Europe, and the United States.

However, the development of resistance to FLT3 inhibitors has limited further improvement in their clinical efficacy.

Therefore, although the discovery of TRKA and FLT3 inhibitors contributes to tumor treatment and greatly saves patients' lives, the inevitable development of drug resistance finally leads to disease progression and limits their clinical application. Gene mutations are one of the causes of drug resistance. Multi-target inhibitors are a new topic in tumor drug development. Studies have shown that multi-target single-entity drugs outperform single-target drugs in terms of efficacy while exhibiting fewer side effects and potentially overcoming resistance. Therefore, the development of new inhibitors against TRKA and FLT3 targets, particularly inhibitors against mutation sites in genes, is of great significance in the treatment of related diseases.

### SUMMARY

The chemical name of chidamide is N-(2-amino-4-fluorophenyl)-4-[N-[(*E*)-3-(pyridin-3-yl)acryloyl]aminomethyl] benzamide. It is a histone deacetylase inhibitor, and its chemical structural formula is shown below:

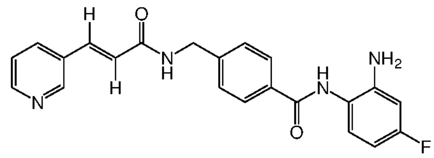

The pharmacological activity of chidamide is described in Chinese Patent No. CN1284772C. Chidamide is a subtype selective histone deacetylase inhibitor that mainly inhibits HDAC1, HDAC2, and HDAC3 among class I HDACs and HDAC10 among class IIb HDACs and can be used to treat diseases associated with abnormal histone deacetylase activity, such as cancers, including lymphomas, solid tumors, hematological tumors, etc.

In 2014, 5-mg chidamide tablets were approved in China for the treatment of peripheral Tcell lymphoma (PTCL). In 2019, 5-mg chidamide tablets were approved in China for the treatment of breast cancer.

Through extensive research, the inventors unexpectedly discovered that, during HPLC analysis of chidamide tablets (chromatography column: $C_{18}$ column, Shimadzu VP-ODS, 5 μm, 4.6 mm × 150 mm; mobile phase: methanol-water-glacial acetic acid (30:70:0.4); detection wavelength: 256 nm; flow rate: 1.0 mL/min), a peak for an unknown substance appeared at a retention time of 18.2 min in the HPLC chromatogram.

Through further investigation, the inventors identified this unknown substance as N-[4-(6-fluoro-1H-2-benzimidazolyl)benzyl]-(E)-3-(pyridin-3-yl)acrylamide. Its structural formula is shown as formula (I):

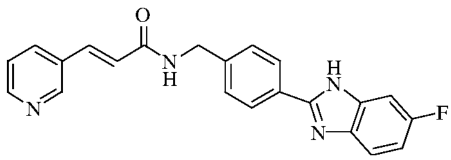

(I).

Chidamide tablets were examined for related substances using an HPLC method with the following conditions: chromatography column: $C_{18}$ column, Shimadzu VP-ODS, 5 μm, 4.6 mm × 150 mm; mobile phase: methanol-water-glacial acetic acid (30:70:0.4); detection wavelength: 256 nm; flow rate: 1.0 mL/min. A control solution of the compound represented by formula (I), N-[4-(6-fluoro-1H-2-benzimidazolyl)benzyl]-(*E*)-3-(pyridin-3-yl)acrylamide, was prepared, its chromatogram was recorded, and the retention time of this substance was determined. A test solution of chidamide tablets was prepared, and its chromatogram was recorded. Based on the retention time of N-[4-(6-fluoro-1H-2-benzimidazolyl)benzyl]-(*E*)-3-(pyridin-3-yl)acrylamide, the content of this substance in chidamide tablets was calculated using the area normalization method.

Through activity studies on this substance, the inventors unexpectedly found that it has excellent inhibitory activity against the kinases TRKA (G667C), FLT3, and FLT3 (D835Y).

Based on the above findings, the present disclosure provides, in a first aspect, a TRKA (G667C), FLT3, and FLT3 (D835Y) target inhibitor compound represented by formula (I) having significant inhibitory effects on the kinases TRKA (G667C), FLT3, and FLT3 (D835Y), N-[4-(6-fluoro-1H-2-benzimidazolyl)benzyl]-(E)-3-(pyridin-3-yl)acrylamide, or a pharmaceutically acceptable salt thereof:

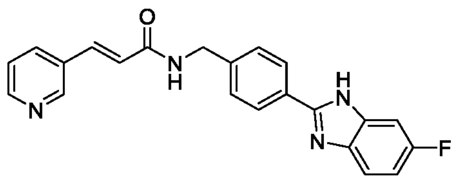

(I) .

The present disclosure provides, in a second aspect, a preparation method for the compound represented by formula (I) described above, wherein the compound is obtained by reacting chidamide with an acid.

In one embodiment, the acid includes inorganic and organic acids; the inorganic acids include hydrochloric acid, sulfuric acid, nitric acid, phosphoric acid, perchloric acid, hydroiodic acid, or hydrobromic acid, and the organic acids include acetic acid, trifluoroacetic acid, lactic acid, succinic acid, fumaric acid, maleic acid, citric acid, benzoic acid, methanesulfonic acid, or p-toluenesulfonic acid.

In one embodiment, the compound represented by formula (I) described above is obtained by reacting chidamide with hydrochloric acid:

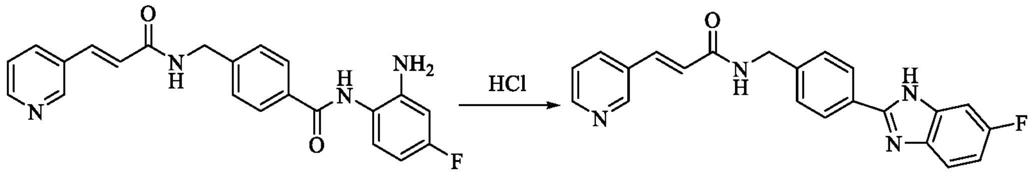


Chidamide (I).

In one embodiment, the compound represented by formula (I) is obtained by performing a reflux reaction of chidamide with 0.2 mol/L hydrochloric acid, then neutralizing with a sodium hydroxide solution, filtering under reduced pressure, washing with water, and drying.

It is noted that the hydrochloric acid and reflux reaction conditions used in the above reaction scheme are exemplary rather than limiting, and that appropriate modifications and adjustments can be made by those skilled in the art.

The present disclosure provides, in a third aspect, a pharmaceutical composition comprising the compound represented by formula (I) or the pharmaceutically acceptable salt thereof described above and a pharmaceutically acceptable carrier.

The present disclosure provides, in a fourth aspect, use of the compound represented by formula (I) or the pharmaceutically acceptable salt thereof described above in the preparation of a medicament for preventing and/or treating diseases associated with TRK and/or FLT3 targets.

In one embodiment, the diseases are diseases associated with TRKA (G667C) and/or FLT3 and/or FLT3 (D835Y) targets.

In one embodiment, the diseases include tumors.

The present disclosure provides, in a fifth aspect, a pharmaceutical composition comprising the compound represented by formula (I) or the pharmaceutically acceptable salt thereof described above and chidamide.

The present disclosure provides, in a sixth aspect, use of the compound represented by formula (I) or the pharmaceutically acceptable salt thereof described above as a standard or a control.

In one embodiment, the present disclosure provides use of the compound represented by formula (I) or the pharmaceutically acceptable salt thereof described above in quality control of chidamide or a formulation comprising chidamide.

In one embodiment, the present disclosure provides use of the compound represented by formula (I) or the pharmaceutically acceptable salt thereof described above in the preparation of a control of chidamide or a formulation comprising chidamide.

In one embodiment, the formulation comprising chidamide is chidamide tablets.

In one embodiment, the present disclosure provides use of the compound represented by formula (I) or the pharmaceutically acceptable salt thereof described above as a control in the examination of chidamide tablets for related substances.

Terms and Definitions:

The term "treating" or "treatment" refers to administering or applying a therapeutic agent to a subject or performing a procedure or modality on a subject for the purpose of obtaining a therapeutic benefit of a disease or health-related condition.

The term "control": In the pharmaceutical field, all researchers know that highly pure compounds can be used as "controls". A control generally refers to a standard substance used for identification, analysis, quantification, and calibration and verification of instrument performance.

The beneficial effects of the present disclosure:

(1) The inventors unexpectedly found that the unknown substance appeared at a retention time of 18.2 min in the HPLC chromatogram of chidamide tablets was the compound represented by formula (I) described above, N-[4-(6-fluoro-1H-2-benzimidazolyl)benzyl]-(E)-3-(pyridin-3-yl)acrylamide. This compound is of a completely new structure, and the inventors prepared it and determined its structure.

(2) The inventors carried out extensive studies on the activity of the compound represented by formula (I), N-[4-(6-fluoro-1H-2-benzimidazolyl)benzyl]-(E)-3-(pyridin-3-yl)acrylamide, and found that it has excellent inhibitory effects on the kinases TRKA (G667C), FLT3, and FLT3 (D835Y), with $IC_{50}$ values of 40.69 nM, 142.6 nM, and 103.8 nM,

respectively. Based on these findings, the present disclosure provides a TRKA (G667C) and FLT3 target inhibitor of a completely new structure and a pharmaceutical composition comprising same.

(3) Through further in-depth research, the inventors found that the compound represented by formula (I), N-[4-(6-fluoro-1H-2-benzimidazolyl)benzyl]-(E)-3-(pyridin-3-yl)acrylamide, exhibits an unpredictable synergistic effect when used in combination with chidamide. Based on this finding, the present disclosure provides a pharmaceutical composition comprising the compound represented by formula (I) described above, N-[4-(6-fluoro-1H-2-benzimidazolyl)benzyl]-(E)-3-(pyridin-3-yl)acrylamide, and chidamide.

(4) The present disclosure provides use of the compound represented by formula (I), N-[4-(6-fluoro-1H-2-benzimidazolyl)benzyl]-(E)-3-(pyridin-3-yl)acrylamide, as a control in the examination of chidamide tablets for related substances to achieve quality control of chidamide.

## BRIEF DESCRIPTION OF THE DRAWINGS

FIG. 1 shows the test results of the effects of the compound N-[4-(6-fluoro-1H-2-benzimidazolyl)benzyl]-(E)-3-(pyridin-3-yl)acrylamide (compound A) and chidamide on the viability of SHP77 cells.

## DETAILED DESCRIPTION OF THE EMBODIMENTS

The present disclosure is further elucidated below with reference to examples. However, the protection scope of the present disclosure is not limited to these examples only. The percentages described in the present disclosure are all weight percentages, unless otherwise indicated. The numerical ranges described in the specification, such as units of measurement or percentages, are intended to provide an unambiguous written reference. One skilled in the art can practice this patent using temperatures, concentrations, amounts, etc. outside these ranges or different from the individual values based on the teachings and principles of the present disclosure, and still obtain the desired results.

Starting materials and experimental instruments:

Chidamide: produced by Shenzhen Chipscreen Pharmaceutical Ltd., batch number: 20220708, purity > 99%.

High performance liquid chromatography: instrument: UltiMate3000; chromatography column: $C_{18}$ column, Shimadzu VP-ODS, 5 μm, 4.6 mm × 150 mm.

Proton nuclear magnetic resonance: instrument: Avance NEO 400; solvent: DMSO-$d_6$.

High-resolution mass spectrometry: instrument: maXis impact.

Infrared spectroscopy: instrument: PerkinElmer Spectrum.

### Example 1: Preparation of Compound N-[4-(6-Fluoro-1H-2-benzimidazolyl) benzyl]-(E)-3-(pyridin-3-yl)acrylamide

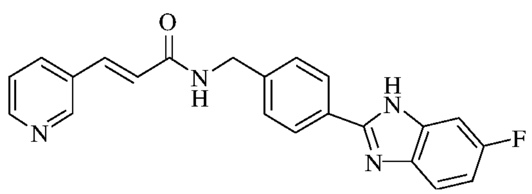

2.0 g of chidamide and 50 mL of 0.2 mol/L hydrochloric acid were added to a reaction flask, and chidamide was ultrasonically dissolved. The mixture was heated at reflux for about 4 h. The reaction mixture was cooled to room temperature, neutralized to a pH of > 12 with a 1 mol/L sodium hydroxide solution, and filtered under reduced pressure. The filter cake was washed with purified water until the filtrate had a pH of 5-7. The solid was collected and dried to give the desired compound (1.6 g, purity (HPLC): 99.9%). HRMS ($M^+$+1) ($C_{22}H_{18}FN_4O$) calculated (%): 373.1420; found (%): 373.1413. $^1$H NMR (DMSO-$d_6$) δ 4.53 (brs, 2H), 6.86 (d, 1H), 7.07 (m, 1H), 7.32-7.68 (m, 6H), 8.03 (d, 1H), 8.15 (d, 2H), 8.58 (d, 1H), 8.80 (br, 2H), 13.03 (brs, 1H). IR ($cm^{-1}$) 3432, 3267, 2917, 1658, 1625, 1537, 1495, 1453, 1423, 1229, 843,

689.

**Example 2: Inhibitory Activity of Compound N-[4-(6-Fluoro-1H-2-benzimidazolyl)benzyl]-(*E*)-3-(pyridin-3-yl)acrylamide (Compound A) Against Kinases TRKA and FLT3 ($IC_{50}$)**

The inhibition of the kinases TRKA (G667C), FLT3, and FLT3 (D835Y) by the compound N-[4-(6-fluoro-1H-2-benzimidazolyl)benzyl]-(E)-3-(pyridin-3-yl)acrylamide of the present disclosure (hereinafter referred to as compound A) was assessed using a homogeneous time-resolved fluorescence (HTRF) method based on biochemical levels.

HTRF is a technology used for detecting a test substance in a pure liquid phase system. Based on two major technologies: fluorescence resonance energy transfer (FRET) and time-resolved fluorescence (TRF), the generated fluorescence intensity was measured using a microplate reader, and the ratio 665 nm/620 nm reflected the extent to which compound A inhibited the activity of the kinases TRKA (G667C), FLT3, and FLT3 (D835Y).

The specific experimental method is shown below:

Reagents and consumables:

| Reagent | Supplier | Cat. No. |
|---|---|---|
| MgCl2 | Sigma | M1028 |
| HTRF KinEASE-TK kit | Cisbio | 62TK0PEC |
| DTT | MCE | HY-15917 |
| DMSO | Sigma | D4540 |
| ATP | Promega | V915B |
| FLT3 | Carna | 08-154 |
| FLT3 D835Y | Proqinase | 0777-0000-1 |
| TRKA G667C | signalchem | N16-12CG-10 |
| Consumable and instrument | Supplier | Cat. No. or model |
| 384 reaction plate | Greiner | 784075 |
| Centrifuge | cence | TDZ5-WS |
| Plate reader | BMG | PHERAstar FSX |

Chidamide was from Shenzhen Chipscreen Pharmaceutical Ltd.

Compound A (N-[4-(6-fluoro-1H-2-benzimidazolyl)benzyl]-(E)-3-(pyridin-3-yl)acrylamide) was from Shenzhen Chipscreen Biosciences Co., Ltd.

**1. Preparation of compound A and kinase reaction buffer**

a) Preparation of compound A or chidamide: The starting stock solution concentration of compound A or chidamide was 20 mM. The stock solution was serially diluted with the solvent 100% DMSO to 0.2 mM, which was 200 times the assay starting concentration.

b) A kinase reaction buffer Assay Buffer was prepared using the following formula:

| Reagent | Concentration |
|---|---|
| 5× Buffer | 1× |
| $MgCl_2$ | 5 mM |
| $MnCl_2$ | 1 mM |
| SEB | 12.5 nM |
| DTT | 1 mM |
| $ddH_2O$ | / |

**2. Preparation of compound A, kinase solution, and substrate mixture for assay use**

a) Compound A or chidamide was diluted with the kinase reaction buffer to 10 concentrations that were 200 times the following final concentrations: 1000 nM, 300 nM, 100 nM, 30 nM, 10 nM, 3 nM, 1 nM, 0.3 nM, 0.1 nM, and 0.03 nM.
b) A TRKA (G667C), FLT3, or FLT3 (D835Y) kinase solution with a concentration that was 2 times the final concentration was prepared using the kinase reaction buffer. A solution of a mixture of ATP and a biotinylated substrate (ATP + TK substrate-biotin polypeptide substrate) with a concentration that was 2 times the final concentration was prepared using the kinase reaction buffer. The final concentrations of the substances at the time of reaction are shown in the table below:

| Kinase | Kinase concentration (nM) | Substrate TK concentration (μM) | ATP concentration (μM) |
|---|---|---|---|
| TRKA G667C | 2 | 1 | 0.5 |
| FLT3 | 0.024 | 1 | 2 |
| FLT3 D835Y | 0.05 | 1 | 0.5 |

**3. HTRF assay**

1) By using an Echo655 liquid dispenser, 25 nL of the diluted compound A or chidamide described above was added to a compound well of a 384 reaction plate, and 25 nL of 100% DMSO was added to each of a negative control well and a positive control well. Subsequently, 2.5 μL of the kinase solution with a concentration that was 2 times the final concentration was added to each of the compound well and the positive control well of the 384 reaction plate, the contents in each of the wells were well mixed, and 2.5 μL of the kinase reaction buffer was added to the negative control well. The plate was incubated at 25 °C for 10 min.

2) 2.5 μL of the substrate mixture with a concentration that was 2 times the final concentration was added to the 384 reaction plate, and the plate was incubated at 25 °C for 50 min.

3) A mixture of XL665-labeled avidin and europium (Eu)-labeled phosphorylated TK antibody with a concentration that was 2 times the final concentration was prepared using the assay buffer, and 5 μL of the above mixture was added to each reaction well. The plate was incubated at 25 °C for 60 min.

4) After the 384-well plate was centrifuged at 1000 rpm for 30 s and shaken for thorough mixing, the fluorescence intensities at 665 nm and 620 nm were measured using EnVision, and the TR-FRET ratio (665 nm/620 nm) was calculated.

**4. Data analysis**

1) The calculation formula for the activity inhibition rate is shown below:

Inhibition rate (%) = 100% - [(TR-FRET ratio_compound) - (TR-FRET ratio_positive control)] / [(TR-FRET ratio_-negative control) - (TR-FRET ratio_positive control)] × 100%

2) Dose-response curve fitting

Dose-response curves were fitted using the log(compound concentration) vs. (dose response - variable slope) of the analysis software GraphPad 7.0 with the log value of the concentration as the X axis and the inhibition rate as the Y axis, and the $IC_{50}$ values for the activity of compound A against the kinases TRKA (G667C), FLT3, and FLT3 (D835Y) were obtained. The results are shown below:

| Compound | Kinase | $IC_{50}$ (nM) |
|---|---|---|
| Compound A | TRKA(G667C) | 40.69 |
| | FLT3 | 142.6 |
| | FLT3(D835Y) | 103.8 |
| Chidamide | TRKA G667C | >1000 |
| | FLT3 | >1000 |
| | FLT3(D835Y) | >1000 |

According to the table above, the compound N-[4-(6-fluoro-1H-2-benzimidazolyl)benzyl]-(E)-3-(pyridin-3-yl) acrylamide (compound A) exhibited significant inhibitory activity against the kinases TRKA (G667C), FLT3, and FLT3 (D835Y), which indicates that the compound is a TRKA (G667C), FLT3, and FLT3 (D835Y) target inhibitor, while chidamide did not exhibit an inhibitory effect on the above kinase targets.

**Example 3: Effect of Combination of Compound N-[4-(6-Fluoro-1H-2-benzimidazolyl)benzyl]-(*E*)-3-(pyridin-3-yl)acrylamide (Compound A) and Chidamide on Viability of SHP77 Cells**

**1. Experimental materials and instruments**

1.1. Materials and sources

The SHP77 cell line was purchased from the American Type Culture Collection (ATCC) and cultured in an RPMI-1640 culture medium (Hyclone) containing 1% dual antibiotics (penicillin-streptomycin, Hyclone) and 10% fetal bovine serum (Gibco).

Chidamide was from Shenzhen Chipscreen Pharmaceutical Ltd.

Compound A (N-[4-(6-Fluoro-1H-2-benzimidazolyl)benzyl]-(*E*)-3-(pyridin-3-yl)acrylamide) and chidamide were both from Shenzhen Chipscreen Biosciences Co., Ltd. The compound and chidamide were dissolved in dimethyl sulfoxide (DMSO, Shanghai Sangon, DN3039A) to prepare 20 mM working mother liquors, and the mother liquors were stored in a freezer at -20 °C before use. When in use, the mother liquors were diluted to prepare working solutions with corresponding final concentrations according to the needs of each experiment.

CellTiter 96® AQueous MTS Reagent Powder (Promega, G1111)

Phenazine methosulfate, PMS (Sigma, P9625)

1.2. Experimental reagents and instruments:

Super clean bench (Airtech)

$CO_2$ cell incubator (RS Biotech, Galaxy S)

Inverted fluorescence microscope (Guangzhou Mshot Technology Co., Ltd.)

Cell counting plate (Shanghai Qiujing Biochemical Reagent Instrument Co., Ltd.)

Tecan infinite F50 absorbance microplate reader

**2. Experimental method**

2.1. Preparation of MTS solution and PMS solution

MTS solution: 42 mg of MTS Reagent Powder was placed in a light shielding container, and 21 mL of PBS was added. The mixture was gently stirred on a magnetic stirrer for 15 min until MTS was completely dissolved. The pH of the

solution was measured. If the pH was higher than 6.5, the pH of the solution was adjusted to 6.5 with 1 M HCl. The solution was sterilized by filtration through a 0.2 μm filter and stored at -20 °C in a dark place.

PMS solution: 1 g of PMS was added to 21.7 mL of PBS to prepare a 150 μM stock solution, and the stock solution was sterilized by filtration, aliquoted, and stored at -20 °C in a dark place.

2.2. Cell seeding: SHP77 cells were seeded in a 96-well cell culture plate at 15,000 cells/180 μL per well, and a BLANK well was set aside. In that well, no cells were added, and only the same volume of the culture medium was added. The plate was incubated at 37 °C with 5% CO2.

2.3. Administration: After seeding and overnight incubation, administration was performed. First, compound A and chidamide were diluted with DMSO to a concentration that was 1000 times the final concentration in each well. Then, the dilutions of compound A and chidamide in DMSO were each diluted into the culture medium in a 1:50 ratio. Compound A was added at a final concentration of 0, 0.625, or 1.25 μM in combination with 0 or 0.5 μM chidamide to 10 μL of the corresponding solution described above.

2.4. Measurement: After 72 h of drug treatment, the 96-well plate was taken out. 20 μL of a mixed solution of MTS and PMS (MTS:PMS = 20:1) was directly added to SHP77 cells. After the plate was incubated for about 1 h, the absorbance at 490 nm in each well was measured using a microplate reader.

2.5. Data processing and analysis

The reading of the BLANK well was $OD_{490\text{-}BLANK}$ (background value). The background value was subtracted from the readings of the wells to obtain the absorbance values of the administration wells, $OD_{490\text{-}T}$, and the absorbance values of the control wells in which the drug concentration was 0, $OD_{490\text{-}T0}$. With the cell viability of the control wells defined as 100%, the relative cell survival rate in each administration well was calculated using the formula:

$$\text{relative cell survival rate (\%)} = (OD_{490\text{-}T} / OD_{490\text{-}T0}) \times 100\%.$$

**3. Experimental results**

The test results of the effects of compound A and chidamide on the viability of SHP77 cells are shown in FIG. 1. After SHP77 cells were treated with compound A alone at final concentrations of 0.625 and 1.25 μM for 72 h, the relative cell survival rates were 77.43 ± 1.18% and 44.09 ± 6.59%, respectively. After cells were treated with chidamide alone at a final concentration of 0.5 μM for 72 h, the relative cell survival rate was 95.45 ± 0.81%. After SHP77 cells were treated with two doses of compound A combined with chidamide, the relative cell survival rates were 38.22 ± 1.92% and 20.01 ± 2.87%, respectively. The above results show that compound A can inhibit tumor cell proliferation in a dose-dependent manner and unexpectedly reveal that the combination of compound A and chidamide can exhibit a significant synergistic inhibitory effect on tumors.

**Example 4: Use of Compound N-[4-(6-Fluoro-1H-2-benzimidazolyl)benzyl]-(*E*)-3-(pyridin-3-yl)acrylamide as Control in Examination of Chidamide Tablets for Related Substances**

**1. Test conditions**

Instrument: UltiMate3000; chromatography column: $C_{18}$ column, Shimadzu VP-ODS, 5 μm, 4.6 mm × 150 mm; mobile phase: methanol-water-glacial acetic acid (30:70:0.4); detection wavelength: 256 nm; flow rate: 1.0 mL/min.

**2. Test method**

(1) About 6 mg of the N-[4-(6-fluoro-1H-2-benzimidazolyl)benzyl]-(*E*)-3-(pyridin-3-yl)acrylamide control (batch No. 000043001-01) was collected, precisely weighed, and placed into a 100-mL measuring flask, and 30 mL of methanol was added. The control was ultrasonically dissolved, and the solution was diluted to volume with water and well shaken. 5 mL of the resulting solution was precisely transferred to a 100-mL measuring flask, diluted to volume with a mixture of solvent methanol and water (30:70), and well shaken. 1 mL of the resulting solution was precisely transferred to a 50-mL measuring flask, diluted to volume with a mixture of solvent methanol and water (30:70), and well shaken. The resulting solution was used as a control solution of N-[4-(6-fluoro-1H-2-benzimidazolyl)benzyl]-(*E*)-3-(pyridin-3-yl)acrylamide. 60 μL of the control solution was precisely collected using a pipette and injected into a liquid chromatograph. The chromatogram was recorded, and the retention time of N-[4-(6-fluoro-1H-2-benzimidazolyl)benzyl]-(*E*)-3-(pyridin-3-yl)acrylamide was measured.

(2) A proper amount of chidamide tablets (batch No. 20220708) was collected, precisely weighed, ground, and well mixed. A proper amount of the mixture (equivalent to about 20 mg of chidamide) was precisely weighed out and placed into a 100-mL measuring flask, and a proper amount of methanol was added. The mixture was ultrasonically dissolved, and the solution was diluted to volume with methanol, well shaken, and filtered. 15 mL of the subsequent filtrate was collected, placed into a 50-mL measuring flask, and diluted to volume with water. The resulting solution was used as a test solution of chidamide tablets. 60 μL of the test solution was precisely measured out and injected into a liquid chromatograph, and the chromatogram was recorded. The content of the substance was calculated using the area normalization method based on the retention time of N-[4-(6-fluoro-1H-2-benzimidazolyl)benzyl]-(*E*)-3-(pyridin-3-yl)acrylamide.

**3. Results**

According to the chromatogram of the control solution of N-[4-(6-fluoro-1H-2-benzimidazolyl)benzyl]-(*E*)-3-(pyridin-3-yl)acrylamide, the retention time of N-[4-(6-fluoro-1H-2-benzimidazolyl)benzyl]-(*E*)-3-(pyridin-3-yl)acrylamide was 18.2 min.

According to the chromatogram of the test solution of chidamide tablets, the content of N-[4-(6-fluoro-1H-2-benzimidazolyl)benzyl]-(*E*)-3-(pyridin-3-yl)acrylamide (retention time: 18.2 min) in chidamide tablets was 0.07%.

The present disclosure has been illustrated using specific examples. However, it is appreciated by those of ordinary skill in the art that the present disclosure is not limited to the specific embodiments. Various changes or modifications can be made by those of ordinary skill in the art within the scope of the present disclosure, and the technical features mentioned in the specification can be combined with one another without departing from the spirit and scope of the present disclosure. Such changes and modifications are all within the scope of the present disclosure.

**Claims**

1. A compound represented by formula (I), N-[4-(6-fluoro-1H-2-benzimidazolyl)benzyl]-(*E*)-3-(pyridin-3-yl)acrylamide, or a pharmaceutically acceptable salt thereof:

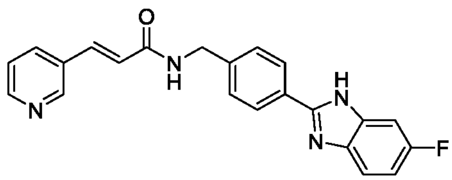

(I) .

2. A preparation method for the compound represented by formula (I) according to claim 1, wherein the compound is obtained by reacting chidamide with an acid.

3. The preparation method according to claim 2, wherein the acid includes inorganic and organic acids; preferably, the compound is obtained by reacting chidamide with hydrochloric acid:

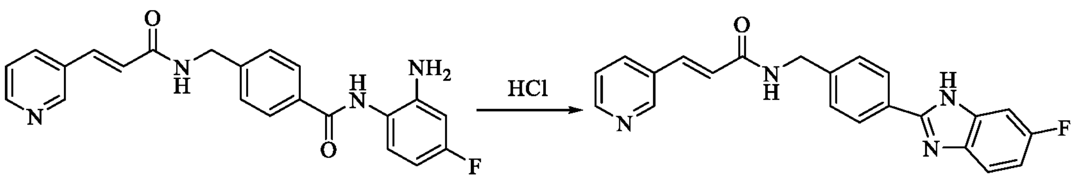


Chidamide (I) .

4. A pharmaceutical composition, comprising the compound represented by formula (I) or the pharmaceutically acceptable salt thereof according to claim 1 and a pharmaceutically acceptable carrier.

**5.** Use of the compound represented by formula (I) or the pharmaceutically acceptable salt thereof according to claim 1 in the preparation of a medicament for preventing and/or treating diseases associated with TRK and/or FLT3 targets.

**6.** The use according to claim 5, wherein preferably, the diseases are diseases associated with TRKA (G667C) and/or FLT3 and/or FLT3 (D835Y) targets; more preferably, the diseases include tumors.

**7.** A pharmaceutical composition, comprising the compound represented by formula (I) or the pharmaceutically acceptable salt thereof according to claim 1 and chidamide.

**8.** Use of the compound represented by formula (I) or the pharmaceutically acceptable salt thereof according to claim 1 as a standard or a control.

**9.** Use of the compound represented by formula (I) or the pharmaceutically acceptable salt thereof according to claim 1 in quality control of a formulation comprising chidamide.

**10.** The use according to claim 9, wherein the formulation comprising chidamide is chidamide tablet.

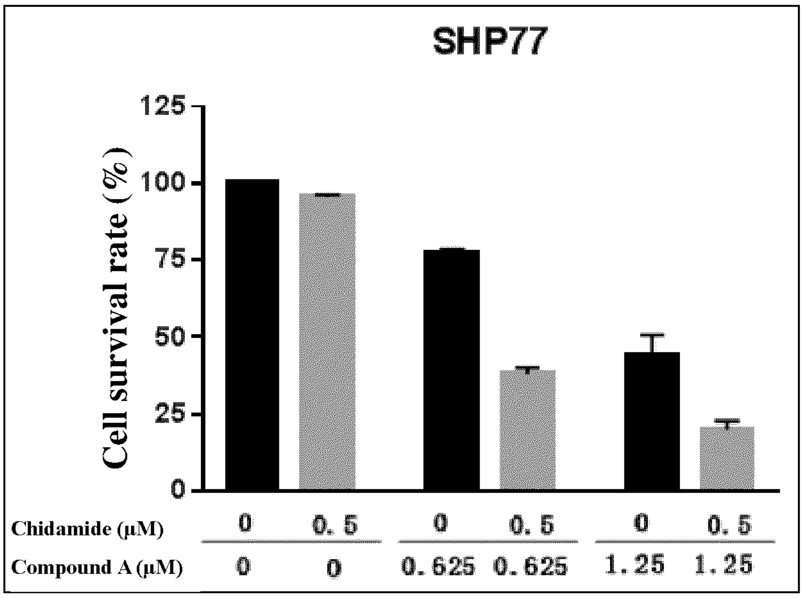
SHP77
Cell survival rate (%)
125
100
75
50
25
0
Chidamide (μM) 0 0.5 0 0.5 0 0.5
Compound A (μM) 0 0 0.625 0.625 1.25 1.25

FIG. 1

**INTERNATIONAL SEARCH REPORT**

International application No.
**PCT/CN2023/076093**

**A. CLASSIFICATION OF SUBJECT MATTER**

C07D401/12(2006.01)i;A61P35/00(2006.01)i;A61K31/4406(2006.01)i;A61K31/4439(2006.01)i;G01N30/02(2006.01)i; G01N30/06(2006.01)i

According to International Patent Classification (IPC) or to both national classification and IPC

**B. FIELDS SEARCHED**

Minimum documentation searched (classification system followed by classification symbols)

IPC: C07D, A61P, A61K, G01N

Documentation searched other than minimum documentation to the extent that such documents are included in the fields searched

Electronic data base consulted during the international search (name of data base and, where practicable, search terms used)

CNTXT, ENTXTC, DWPI, CNKI, STN: 微芯, 西达本胺, 西达苯胺, chidamide, FLT3, TRKA, 式I结构式, formula I structural formula

**C. DOCUMENTS CONSIDERED TO BE RELEVANT**

| Category* | Citation of document, with indication, where appropriate, of the relevant passages | Relevant to claim No. |
|---|---|---|
| PX | CN 115322171 A (SHENZHEN CHIPSCREEN BIOSCIENCES CO., LTD.) 11 November 2022 (2022-11-11)<br>claims 1-10, and embodiments 1-4 | 1-10 |
| A | CN 112386593 A (SHENZHEN CHIPSCREEN BIOSCIENCES CO., LTD.) 23 February 2021 (2021-02-23)<br>claims 1-18, and embodiments 1-2 | 1-10 |
| A | CN 103880736 A (SHENZHEN CHIPSCREEN BIOSCIENCES CO., LTD.) 25 June 2014 (2014-06-25)<br>claims 1-11, and embodiments 1-18 | 1-10 |
| A | CN 109223782 A (CHINA PHARMACEUTICAL UNIVERSITY) 18 January 2019 (2019-01-18)<br>claims 1-4, and embodiments | 1-10 |
| A | CN 112274509 A (WEST CHINA HOSPITAL, SICHUAN UNIVERSITY) 29 January 2021 (2021-01-29)<br>claims 1-7, and embodiments 1-7 | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 May 2023** | **29 May 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/076093**

**C. DOCUMENTS CONSIDERED TO BE RELEVANT**

| Category* | Citation of document, with indication, where appropriate, of the relevant passages | Relevant to claim No. |
|---|---|---|
| A | CN 112439070 A (SHENZHEN CHIPSCREEN BIOSCIENCES CO., LTD.) 05 March 2021 (2021-03-05)<br>claims 1-12, and embodiments 1-8 | 1-10 |
| A | CN 114786672 A (GNT BIOTECH & MEDICALS CORPORATION) 22 July 2022 (2022-07-22)<br>claims 1-32, and examples 1-10 | 1-10 |
| A | CN 113908159 A (SHENZHEN CHIPSCREEN BIOSCIENCES CO., LTD. et al.) 11 January 2022 (2022-01-11)<br>claims 1-10, and embodiments 1-4 | 1-10 |
| A | WO 2021219040 A1 (GNT BIOTECH & MEDICALS CORPORATION) 04 November 2021 (2021-11-04)<br>claims 1-20, and embodiments, and tables 1-14 | 1-10 |
| A | 张祥娜等 (ZHANG, Xiangna et al.). "西达本胺衍生物的设计合成及抗肿瘤活性评价 (Design, Synthesis and Evaluation of Anti-tumor Activities of Chidamide Derivatives)"<br>*有机化学 (C鱂nese Journal of Organic Chemistry),*<br>Vol. 39, No. 7, 01 April 2019 (2019-04-01), 1983-1989<br>entire document | 1-10 |

Form PCT/ISA/210 (second sheet) (July 2022)

**INTERNATIONAL SEARCH REPORT**
**Information on patent family members**

International application No.
**PCT/CN2023/076093**

| Patent document cited in search report | Publication date (day/month/year) | Patent family member(s) | Publication date (day/month/year) |
|---|---|---|---|
| CN 115322171 A | 11 November 2022 | CN 115322171 B | 17 March 2023 |
| CN 112386593 A | 23 February 2021 | TW 202114666 A | 16 April 2021 |
| | | TWI 741731 B | 01 October 2021 |
| | | WO 2021027912 A1 | 18 February 2021 |
| CN 103880736 A | 25 June 2014 | TW 201538485 A | 16 October 2015 |
| | | TWI 577662 B | 11 April 2017 |
| | | WO 2015149435 A1 | 08 October 2015 |
| | | CN 106916100 B | 15 September 2020 |
| CN 109223782 A | 18 January 2019 | None | |
| CN 112274509 A | 29 January 2021 | CN 112274509 B | 26 April 2022 |
| CN 112439070 A | 05 March 2021 | WO 2021037128 A1 | 04 March 2021 |
| | | TW 202110433 A | 16 March 2021 |
| CN 114786672 A | 22 July 2022 | WO 2021046765 A1 | 18 March 2021 |
| | | CA 3153597 A1 | 18 March 2021 |
| | | AU 2019465985 A1 | 28 April 2022 |
| | | KR 20220061179 A | 12 May 2022 |
| | | BR 112022004471 A2 | 31 May 2022 |
| | | EP 4028002 A1 | 20 July 2022 |
| | | JP 2022547555 W | 14 November 2022 |
| CN 113908159 A | 11 January 2022 | WO 2022007745 A1 | 13 January 2022 |
| | | TW 202214242 A | 16 April 2022 |
| WO 2021219040 A1 | 04 November 2021 | EP 4142733 A1 | 08 March 2023 |
| | | BR 112022022015 A2 | 13 December 2022 |
| | | KR 20230005939 A | 10 January 2023 |
| | | US 2021379032 A1 | 09 December 2021 |
| | | CA 3176748 A1 | 04 November 2021 |
| | | AU 2021265994 A1 | 15 December 2022 |
| | | IL 297687 A | 01 December 2022 |
| | | TW 202200557 A | 01 January 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 1284772 C